# EUROPÄISCHE PATENTSCHRIFT

(11) **EP 3 668 427 B1**
(45) Veröffentlichungstag und Bekanntmachung des Hinweises auf die Patenterteilung: **28.10.2026**
(21) Anmeldenummer: 18752488.9
(22) Anmeldetag: 09.08.2018
(51) Int. Cl.: A61B 17/86, A61B 17/88, A61B 17/72

(54) **KNOCHENSCHRAUBE**
BONE SCREW
VIS À OS

(30) Priorität: 14.08.2017 AT 506722017
(43) Veröffentlichungstag der Anmeldung: 24.06.2020
(73) Patentinhaber: Surgebright GmbH, 4040 Lichtenberg (AT)
(72) Erfinder: PASTL, Klaus, 4040 Lichtenberg (AT)
(74) Vertreter: KLIMENT & HENHAPEL
(86) Internationale Anmeldenummer: PCT/EP2018/071619
(87) Internationale Veröffentlichungsnummer: WO 2019/034522

(56) Entgegenhaltungen:
- WO-A1-00/66011
- US-A- 6 045 554
- US-A1- 2001 007 074
- US-A1- 2012 029 577
- US-B1- 7 182 781

## Beschreibung

Die Erfindung betrifft ein Knochentransplantat aus einem kortikalen Knochenmaterial mit einem Schraubenschaft, der mit einem Außengewinde versehen ist, sowie einem Schraubenkopf zur Einleitung eines Eindrehmoments, wobei der Schraubenkopf eine um eine Schraubenkopfachse rotationssymmetrische Außenmantelfläche aufweist, die mit einem Außengewinde versehen ist, sowie zumindest zwei um die Schraubenkopfachse verteilt angeordnete, in Richtung der Schraubenkopfachse axial verlaufende und in die Stirnfläche des freien Endes des Schraubenkopfes mündende Ausnehmungen zur Aufnahme eines Eindrehwerkzeuges aufweist, wobei die Ausnehmungen jeweils durch sich von der Außenmantelfläche in Richtung der Schraubenkopfachse erstreckende Seitenflächen gebildet werden, die in einem konkav ausgeführten achsnahen Flächenabschnitt ineinander übergehen, dadurch gekennzeichnet, dass die von der Außenmantelfläche in Richtung der Schraubenkopfachse verlaufenden Seitenflächen der Ausnehmungen konvex ausgeführt sind, gemäß Anspruch 1.

Eine gattungsgemäße Schraube wurde in der US 7 182 781 B1 beschrieben. Weitere Ausführungen von Knochenschrauben wurden in der US 6 045 554 A und der WO 00/66011 A1 beschrieben. Schrauben für die chirurgisch operative Osteosynthese werden in herkömmlicher Weise aus Metall bzw. Metalllegierungen gefertigt. Ferner sind Schrauben aus resorbierbarem Material, etwa Polyglykolid und Polylaktid, bekannt. Unterschiedliche Ausführungen von Schrauben aus einem resorbierbaren Material wurden etwa in der US 2001/007074 A1 und der US 2012/029577 A1 beschrieben. Schrauben dieser Art weisen in der chirurgischen Praxis allerdings mehrere Nachteile auf. So müssen etwa Schrauben aus Metall bzw. Metalllegierungen einerseits durch eine Zweitoperation wieder entfernt werden, und unterliegen andererseits Veränderungen durch Korrosion. Damit erhöhen sich die Kosten im Gesundheitssystem, sowie die gesundheitlichen Risiken für jeden Patienten durch eine neuerliche Operation. Bei den meisten Knochenfrakturen erfolgt die operative Versorgung mit Platte und einer Vielzahl an Schrauben, die später wieder entfernt werden müssen.

Sämtliche resorbierbaren Materialien im menschlichen oder tierischen Körper wiederum bilden zwar je nach Material eine mehr oder weniger feste Brücke zwischen den zu osteosynthetisierenden Knochen, werden aber aufgelöst, was die Festigkeit der Osteosynthese der betroffenen Knochen negativ beeinflusst. Des Weiteren führen manche resorbierbare Synthesematerialien während ihres Abbaus zu großen Osteolysen in dem umgebenden Knochen, also zu einem Wegweichen des Empfängerknochens von der Schraube.

Schrauben aus allogenem Knochen (Femur und Tibia-Corticalis) verfügen hingegen über mehrere Vorteile. Sie werden ohne Abstoßungsreaktion vaskularisiert und umgebaut, und sind vor allem für Osteosynthesen dort geeignet, wo kleine Knochenfragmente zusammengefügt werden müssen, da durch die Schraube bereits bei der Operation eine tragende Knochenbrücke entsteht, die sich vom Zeitpunkt der Operation an verbessert, indem sie sich umbaut und voll in den lebenden Knochen integriert und eingebaut wird. Schrauben mit einem Durchmesser von 3-4 mm etwa werden innerhalb von 2 Monaten vollständig mit Gefäßen durchwachsen. Diese Knochenschrauben können daher auch als Knochentransplantate bezeichnet werden. Im Gegensatz dazu stellen Metallschrauben eher ein Hindernis für die Knochenneubildung dar, insbesondere verringern sie durch deren bloße Präsenz die zur Verfügung stehende Oberfläche, die für die Knochenheilung vorhanden wäre. Abbaubare Materialien wiederum haben ihre maximale Festigkeit zum Zeitpunkt der Operation. Für sie gelten dieselben Nachteile wie für die Metallschrauben, des Weiteren nimmt die Festigkeit rapide ab, sobald der Abbauprozess eintritt, wodurch die zu osteosynthetisierende Knochenstelle zumindest vorübergehend wieder eine Schwächung erfährt.

Des Weiteren entfällt bei Knochenschrauben aus allogenem Knochen eine Zweitoperation zur Entfernung des Osteosynthesematerials, da der Knochen vollständig in eigenen Knochen umgewandelt (nicht resorbiert!) wird. Für den Patienten verringert sich somit das Operationsrisiko, für das Gesundheitssystem verringern sich gezwungenermaßen die Kosten. Schrauben aus allogenem Knochen stören auch nicht bei der Anwendung bildgebender Verfahren, im Gegensatz zu Metallschrauben, die störende Artefakte im MRI und CT hinterlassen. Auch Nachuntersuchungen sind problemlos möglich, und lassen eine bessere Beurteilung des Heilungserfolges zu. Daher eignen sich Schrauben aus Knochenmaterial auch dazu im Knochen zur Gänze versenkt zu werden, etwa auch im Rahmen einer intramedullären Platzierung der Schraube in der Markhöhle beispielsweise von Röhrenknochen.

Allerdings muss bei der Anwendung von Schrauben aus allogenem Knochen in der chirurgischen Praxis beachtet werden, dass sie sich hinsichtlich Eindrehwiderstand und Festigkeit erheblich von Metallschrauben unterscheiden. Da sie aus allogener, humaner Corticalis gewonnen werden, ist nicht zu erwarten, dass Kenntnisse zu Gewindeformen, Eindrehwiderstand oder Festigkeit wie sie von Schrauben aus Metall bekannt sind, ohne weiteres übertragbar sind. Tatsächlich besteht hierin auch ein Grund dafür, dass Schrauben aus autologem oder allogenem Knochen bislang in der chirurgischen Praxis noch nicht eingesetzt wurden.

Zudem erweist es sich bei Schrauben aus kortikalem Knochenmaterial ungleich schwieriger Zug- oder Kompressionsschrauben insbesondere mit selbstschneidendem Gewinde herzustellen. Zug- oder Kompressionsschrauben ziehen im Zuge des Einschraubens die beiden zu verbindenden Knochenteile zueinander. Der Operateur setzt hierzu in eine vorgefertigte Bohrung die Schraube ein, die in vorteilhafter Weise über ein selbstschneidendes Gewinde verfügt, wobei die Schraube nach Durchtritt durch den ersten Knochenteil und Eintritt in den zweiten Knochenteil beide Knochenteile aneinander presst. Falls die Schraube über kein selbstschneidendes Gewinde verfügt, muss in die vorgefertigte Bohrung zunächst ein Gewinde vorgeschnitten werden, bevor die Schraube eingedreht werden kann. Die Anwendung von Schrauben aus Knochenmaterial fand nicht zuletzt auch deshalb bislang noch wenig Verbreitung, da die Anfertigung von Zug- oder Kompressionsschrauben insbesondere mit selbstschneidendem Gewinde als unmöglich galt, weil das erforderliche Eindrehmoment nicht gewährleistet werden könne.

Es ist somit das Ziel der Erfindung Schrauben aus kortikalem Knochen so zu verbessern, dass das maximal mögliche Eindrehmoment vergrößert wird und eine optimale Festigkeit der Knochenverbindung erreicht wird. Insbesondere sollen Anwendungen wie die Herstellung einer Zug- oder Kompressionsschraube, oder die Herstellung einer intramedullären Schienung erleichtert werden.

Diese Ziele werden durch die Merkmale von Anspruch 1 erreicht. Anspruch 1 bezieht sich auf ein Knochentransplantat aus einem kortikalen Knochenmaterial mit einem Schraubenschaft, der mit einem Gewinde versehen ist, sowie einem Schraubenkopf zur Einleitung eines Eindrehmoments, wobei der Schraubenkopf eine um eine Schraubenkopfachse rotationssymmetrische Außenmantelfläche aufweist, die mit einem Außengewinde versehen ist, sowie zumindest zwei um die Schraubenkopfachse verteilt angeordnete, in Richtung der Schraubenkopfachse axial verlaufende und in die Stirnfläche des freien Endes des Schraubenkopfes mündende Ausnehmungen zur Aufnahme eines Eindrehwerkzeuges aufweist, wobei die Ausnehmungen jeweils durch sich von der Außenmantelfläche in Richtung der Schraubenkopfachse erstreckende Seitenflächen gebildet werden, die in einem konkav ausgeführten achsnahen Flächenabschnitt ineinander übergehen. Erfindungsgemäß wird hierbei vorgeschlagen, dass die von der Außenmantelfläche in Richtung der Schraubenkopfachse verlaufenden Seitenflächen der Ausnehmungen konvex ausgeführt sind.

Erfindungsgemäß ist somit auch der Schraubenkopf mit einem Außengewinde versehen und trägt somit zur Festigkeit der Knochenverbindung bei. Insbesondere kann auch der Schraubenkopf beispielsweise im Rahmen einer intramedullären Schienung in den Knochen eingedreht werden ohne abgeschnitten werden zu müssen. Das Außengewinde wird lediglich von den erfindungsgemäß zur Einleitung eines Eindrehmoments vorgesehenen, axial verlaufenden Ausnehmungen unterbrochen, deren Seitenflächen sich von der Außenmantelfläche in Richtung der Schraubenkopfachse erstrecken und in einem achsnahen Flächenabschnitt ineinander übergehen. Das Knochentransplantat ist ansonsten bohrungsfrei und besteht zur Gänze aus Knochenmaterial. Insbesondere im Bereich der Schraubenkopfachse verbleibt somit Knochenmaterial, es werden lediglich axiale Ausnehmungen in den Außenmantel des Schraubenkopfes eingefräst, die in die proximale Stirnfäche des Schraubenkopfes münden. Die axiale Länge der axialen Ausnehmungen ist dabei wertmäßig zumindest so groß wie der Durchmesser des Schraubenkopfes in diesem Bereich. In diese Ausnehmungen können axiale Fortsätze eines Eindrehwerkzeuges stirnseitig axial eingeschoben werden. Das Eindrehmoment wird in weiterer Folge auf die Seitenflächen der Ausnehmungen ausgeübt. Aufgrund der erfindungsgemäßen Ausführung des Schraubenkopfes kann das maximale Eindrehmoment jedoch erhöht werden, da die Festigkeit des Schraubenkopfes durch Wegnahme einer geringstmöglichen Menge an Knochenmaterial erhöht wird. Zudem wird das Eindrehmoment auf kinematisch günstige Weise im äußeren Umfangsbereich des Schraubenkopfes eingebracht. Das maximale Eindrehmoment kann auf diese Weise ausreichend erhöht werden, um auch eine Anwendung von Knochenschrauben als Zug- oder Kompressionsschrauben zu ermöglichen. Zudem konnte der Anmelder zeigen, dass sogar die Ausführung eines selbstschneidenden Gewindes zur Anwendung im spongiösen Knochenbereich möglich ist und die Knochenschraube aus kortikalem Knochenmaterial somit in der Lage ist im spongiösen Knochen verwendet zu werden, ohne vorher ein Gewinde im spongiösen Knochen vorschneiden zu müssen.

Das Eindrehmoment wird erfindungsgemäß zusätzlich erhöht, indem die von der Außenmantelfläche in Richtung der Schraubenkopfachse verlaufenden Seitenflächen der Ausnehmungen konvex ausgeführt sind und der achsnahe Flächenabschnitt konkav ausgeführt ist. Auf diese Weise können die Kontaktfläche für die Fortsätze des Eindrehwerkzeuges sowie die verbleibende Knochenmenge im Schraubenkopf erhöht werden. Beides erhöht das Eindrehmoment. Zudem wird dadurch auch im Schraubenkopfbereich die Knochenoberfläche vergrößert und das Einwachsen in den Lagerknochen verbessert.

Weiters wird zur Erhöhung des Eindrehmoments vorgeschlagen, dass vier symmetrisch um die Schraubenkopfachse verteilt angeordnete Ausnehmungen vorgesehen sind. Im Zuge des axialen Einführens der Fortsätze des Eindrehwerkzeugs werden im Fall von vier symmetrisch um die Schraubenkopfachse verteilt angeordneten Ausnehmungen bei den für Knochenschrauben üblichen Durchmessern im einstelligen Millimeterbereich die zwischen den Ausnehmungen verbleibenden Schraubenkopfbereiche von den Fortsätzen des Eindrehwerkzeuges umgriffen und gewissermaßen "eingespannt", wodurch ein Abbrechen von Knochenmaterial unterbunden wird.

Des Weiteren wird gemäß einer bevorzugten Ausführungsform vorgeschlagen, dass die Ausnehmungen an ihrem der Stirnfläche abgewandten axialen Ende einen Bereich aufweisen, in dem sich die in radialer Richtung gemessene Tiefe der Ausnehmungen stetig verringert. Werden axiale Fortsätze eines Eindrehwerkzeuges axial in die Ausnehmungen eingeführt, bilden diese Bereiche verringerter Tiefe zusätzlich zu dem zwischen Ausnehmungen und Fortsätzen gebildeten Formschluss einen Reibschluss zu den Fortsätzen, wodurch die Kopplungskräfte erhöht werden. Auf diese Weise wird auch ein endoskopischer oder arthroskopischer Einsatz der Schraube ermöglicht. Ein weiterer Vorteil dieser Ausführung besteht darin, dass die Herstellung der Knochenschraube sehr erleichtert wird, da sie im Zuge der Herstellung sterilisiert werden muss und dabei eine geringe Schrumpfung erfährt, die den Formschluss zum Eindrehwerkzeug beeinträchtigen könnte. Aufgrund des zusätzlichen Reibschlusses können hohe Kopplungskräfte gewährleistet werden, auch wenn das Knochenmaterial im Zuge der Sterilisierung eine Schrumpfung erfahren hat.

Zur Ausführung einer Zug- oder Kompressionsschraube wird vorgeschlagen, dass das über Schraubenschaft und Schraubenkopf verlaufende Außengewinde zwei Abschnitte unterschiedlicher Gewindesteigung aufweist. Diese beiden Abschnitte liegen jeweils in den zu verbindenden Knochenbereichen. Aufgrund der unterschiedlichen Gewindesteigung werden bei einem bestimmten Drehwinkel, der freilich für beide Abschnitte gleich ist, die zu verbindenden Knochenbereiche aneinander gepresst.

Zur Ausführung einer Schraube mit selbstschneidendem Gewinde wird vorgeschlagen, dass das über Schraubenschaft und Schraubenkopf verlaufende Außengewinde zwei Abschnitte mit unterschiedlichem Gewindeaußendurchmesser aufweist. Der Schraubenkopf liegt dabei im Bereich mit dem größeren Gewindeaußendurchmesser. Bei einer solchen Schraube kann im Zuge der operativen Osteosynthese zunächst ein Kernloch für den größeren Gewindeaußendurchmesser in einem ersten Knochenteil vorgefertigt werden, und durch das größere Kernloch hindurch ein Kernloch für den geringeren Gewindeaußendurchmesser in einem zweiten Knochenteil, der mit dem ersten Knochenteil verbunden werden soll. Die Schraube kann nun ohne vorher ein Gewinde in das jeweilige Kernloch einschneiden zu müssen mit dem Abschnitt mit dem geringeren Gewindeaußendurchmesser durch das Kernloch für den größeren Gewindeaußendurchmesser eingeführt werden, bis es in das Kernloch des zweiten Knochenteils eingedreht werden kann. In weiterer Folge tritt die Schraube mit ihrem Abschnitt mit größerem Gewindeaußendurchmesser in das Kernloch des ersten Knochenteils ein und schneidet sich dort selbsttätig das Gewinde.

Aufgrund der erfindungsgemäßen Ausführung des Schraubenkopfes wird die Einleitung des Eindrehmoments optimiert, also die Übertragung des über ein Eindrehwerkzeug auf den Schraubenkopf ausgeübten Drehmoments. Die erfindungsgemäßen Ausnehmungen gestatten dabei auch ein Fluchten des äußeren Schraubenkopfdurchmessers mit dem äußeren Durchmesser des Schraubenschaftes sowie mit dem äußeren Durchmesser des Eindrehwerkzeuges und ermöglichen dadurch auch neue chirurgische Einsatzgebiete wie beispielsweise bei der intramedullären Schienung oder beim arthroskopischen Einsatz. Ein großer Vorteil besteht zudem darin, dass das Schraubentransplantat in jeder beliebigen Knochentiefe versenkt werden kann. Der Gewindeaußendurchmesser des Knochentransplantats liegt dabei vorzugsweise zwischen 7,0mm und 4,5mm. Die Länge des Knochentransplantats ist dabei zumindest dreimal so groß wie der Durchmesser des Knochentransplantats im Fall einer zylindrischen Ausführung und zumindest dreimal so groß wie der größte Durchmesser des Knochentransplantats im Fall einer kegelstumpfförmigen Ausführung des Knochentransplantats.

Das Knochentransplantat kann je nach chirurgischer Anwendung zylindrisch oder kegelstumpfförmig ausgeführt sein. Eine kegelstumpfförmige Ausführung wird etwa bei einer Ausführung des Knochentransplantats als Fadenanker vorteilhaft sein. Fadenanker werden zur Refixation von Weichteilgewebe, insbesondere zur Refixation von Sehnen an Knochen verwendet. Medizinische Fadenanker weisen üblicherweise einen Ankerkörper auf, der zur Befestigung des Fadenankers in einen Knochen eingetrieben wird. Hierzu ist an dem Ankerkörper ein Gewinde ausgebildet, sodass der Fadenanker in den Knochen einschraubbar ist. Darüber hinaus weisen diese Fadenanker zumindest eine Fadenführung auf, die als Aufnahme und Führung für einen Faden dient, mittels dem das Weichteilgewebe an dem Fadenanker und somit auch an dem Knochen befestigt wird. In herkömmlicher Weise wird dabei die Fadenführung von einer sich quer zur Längsachse des Fadenankers erstreckenden Durchbrechung gebildet. In diese Durchbrechung werden ein oder mehrere Fäden derart eingefädelt, dass die beiden Enden eines Fadens an den beiden Öffnungen der Durchbrechung außenseitig der Umfangsseite des Fadenankers proximalwärts geführt sind. Herkömmliche Fadenanker werden dabei aus Titan oder bioresorbierbare Materialien wie Polylactid gefertigt und zeigen im praktischen Einsatz große Nachteile, insbesondere aufgrund von osteolytischen Prozessen und Entzündungsprozessen. Osteolytische Prozesse können zu einer Lockerung und schließlich zu einem Ausbrechen des Implantats führen. Die Bioresorption wiederum ist ein jahrelanger Prozess, der von Entzündungen und Schmerzen begleitet sein kann. Bei Verwendung eines erfindungsgemäßen Knochentransplantats als Fadenanker werden solche Nachteile vermieden. Hierfür wird zunächst vorgeschlagen, dass der Schraubenschaft auf seinem dem Schraubenkopf abgewandten, freien Ende eine zur Schraubenkopfachse senkrechte Stirnfläche aufweist. Bei diesem, dem Schraubenkopf abgewandten, freien Ende handelt es sich somit um das distale Ende der Schraube, das in der chirurgischen Anwendung in eine vorgefertigte Bohrung eingeführt wird. Des Weiteren wird vorgeschlagen, dass diese zur Schraubenkopfachse senkrechte Stirnfläche des Schraubenschaftes mit einer die Stirnfläche querenden Nut versehen ist, die an ihren gegenüberliegenden Seiten jeweils in die Außenmantelfläche des Schraubenschaftes mündet. Diese Nut stellt die Fadenführung dar, in die bei der chirurgischen Anwendung als Fadenanker ein Faden eingelegt und an der Außenmantelfläche entlang proximalwärts geführt werden kann, bevor das Knochentransplantat in die vorgefertigte Bohrung mithilfe eines Eindrehwerkzeuges eingedreht wird. Hierfür weist das Knochentransplantat an seinem proximalen Ende den Schraubenkopf mit den erfindungsgemäßen Ausnehmungen zur Aufnahme des Eindrehwerkzeuges auf.

Das erfindungsgemäße Knochentransplantat kann in ähnlicher Weise auch als Sehnenanker zur Refixation von Sehnen an Knochen verwendet werden. Hierbei wird in eine vorgefertigte Bohrung das erfindungsgemäße Knochentransplantat mithilfe eines Eindrehwerkzeuges eingedreht, wobei das Sehnenende durch Reibschluss zwischen der vorgefertigten Bohrung und dem eingesetzten Knochentransplantat befestigt wird. Erste Versuche der Anmelderin zeigen eine gegenüber herkömmlichen Sehnenankern überlegene Festigkeit des Sehnenankers. Das erfindungsgemäße Knochentransplantat ist hierfür zäpfchenförmig ausgeführt und weist somit proximal einen zylindrischen Abschnitt auf, der distal in einen konischen Abschnitt übergeht. Das Außengewinde erstreckt sich dabei zumindest über den zylindrischen Abschnitt. An seinem proximalen Ende weist der zylindrische Abschnitt den Schraubenkopf mit den erfindungsgemäßen Ausnehmungen zur Aufnahme des Eindrehwerkzeuges auf.

Die Erfindung wird Im Folgenden anhand von Ausführungsbeispielen mithilfe der beiliegenden Figuren näher erläutert. Hierbei zeigen die
Fig. 1 eine Ausführungsform eines erfindungsgemäßen Knochentransplantats,
Fig. 2 das Detail Ader Fig. 1,
Fig. 3 einen Querschnitt entlang der Schnittebene B-B der Fig. 2,
Fig. 4 eine Ausführungsform einer weiteren Ausführungsform eines erfindungsgemäßen Knochentransplantats,
Fig. 5 das Detail A der Fig. 4,
Fig. 6 das Detail C der Fig. 4,
Fig. 7 einen Querschnitt entlang der Schnittebene B-B der Fig. 5,
Fig. 8 eine Seitenansicht einer Ausführungsform eines Eindrehwerkzeuges für ein erfindungsgemäßes Knochentransplantat,
Fig. 9 das Detail A der Fig. 8, und die
Fig. 10 einen Querschnitt entlang der Schnittebene B-B der Fig. 9.

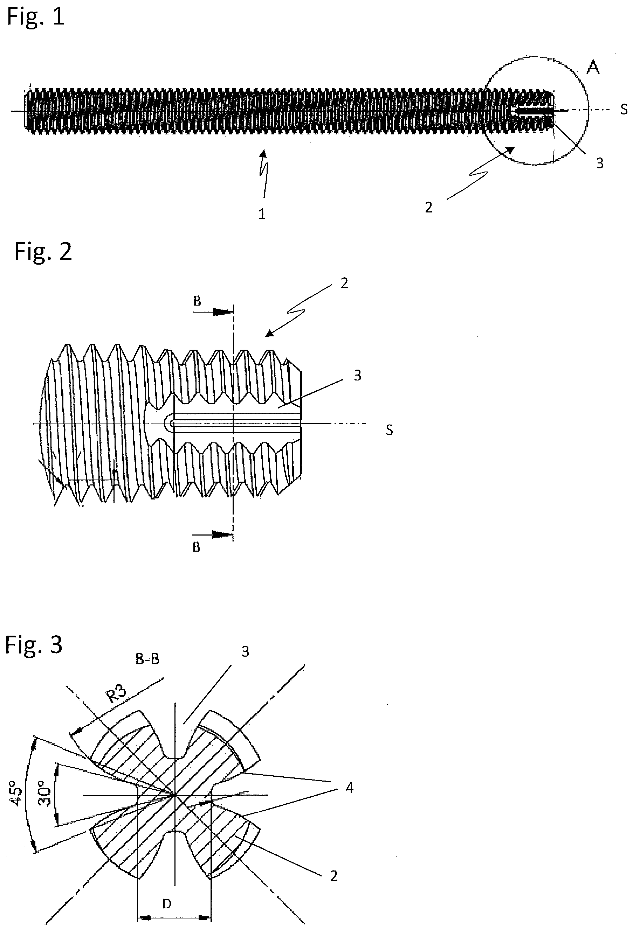

Zunächst wird auf die Fig. 1-3 eingegangen, die eine erste Ausführungsform eines Knochentransplantats aus einem kortikalen Knochenmaterial für die chirurgisch operative Osteosynthese zeigen. Das Knochentransplantat weist einen zylindrischen Schraubenschaft 1 auf, der mit einem Außengewinde versehen ist, sowie einen Schraubenkopf 2 zur Einleitung eines Eindrehmoments, der auch einheilt und nicht weggeschnitten werden muss wie herkömmliche Schraubenköpfe. Der Schraubenkopf 2 weist ferner eine um eine Schraubenkopfachse S rotationssymmetrische Außenmantelfläche auf, die ebenfalls mit einem Außengewinde versehen ist, sowie vier um die Schraubenkopfachse S verteilt angeordnete, in Richtung der Schraubenkopfachse S axial verlaufende und in die Stirnfläche des freien Endes des Schraubenkopfes 2 mündende Ausnehmungen 3 zur Aufnahme eines Eindrehwerkzeuges (siehe Fig. 2 und 3). Die axial verlaufenden Ausnehmungen 3 werden jeweils durch sich von der Außenmantelfläche in Richtung der Schraubenkopfachse S erstreckende Seitenflächen 4 gebildet, die in einem achsnahen Flächenabschnitt ineinander übergehen. (siehe Fig. 3). Die Ausnehmungen 3 weisen dabei an ihrem der Stirnfläche abgewandten axialen Ende einen Bereich auf, in dem sich die in radialer Richtung gemessene Tiefe der Ausnehmungen 3 stetig verringert (siehe Fig. 2). Wie insbesondere der Fig. 3 zu entnehmen ist, sind die von der Außenmantelfläche in Richtung der Schraubenkopfachse S verlaufenden Seitenflächen 4 der Ausnehmungen 3 konvex ausgeführt sind, und der achsnahe Flächenabschnitt konkav.

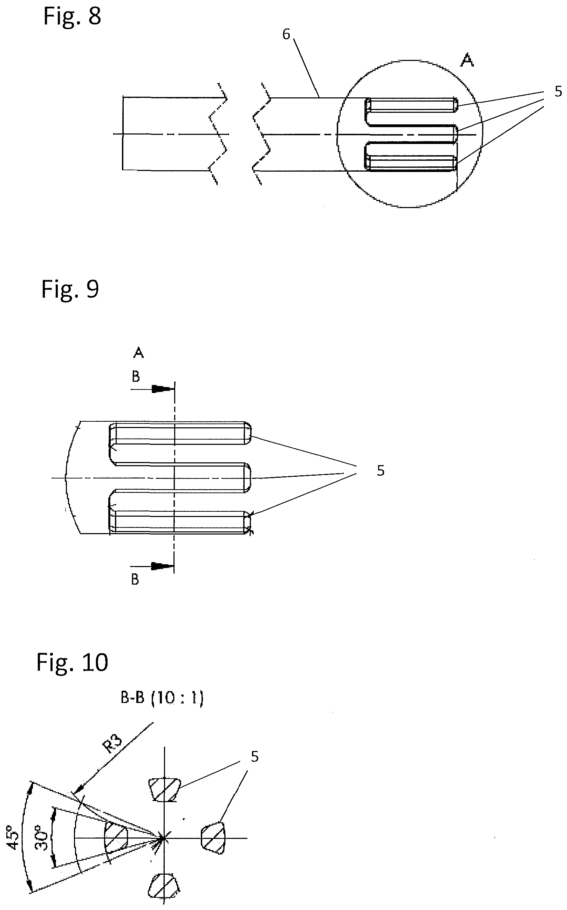

Wie den Fig. 1-3 zu entnehmen ist, erstreckt sich das Außengewinde mit unveränderten Gewindeparametern sowohl über den Schraubenschaft 1, als auch über den Schraubenkopf 2. Der Schraubenkopf 2 trägt somit zur Festigkeit der Knochenverbindung bei. Insbesondere kann auch der Schraubenkopf 2 beispielsweise im Rahmen einer intramedullären Schienung in den Knochen eingedreht werden ohne abgeschnitten werden zu müssen. Das Außengewinde wird lediglich von den zur Einleitung eines Eindrehmoments vorgesehenen, axial verlaufenden Ausnehmungen 3 unterbrochen. Im Bereich der Schraubenkopfachse S verbleibt somit Knochenmaterial mit einem Kerndurchmesser D, wie in der Fig. 3 eingezeichnet ist, da lediglich axiale Ausnehmungen 3 in den Außenmantel des Schraubenkopfes 2 eingefräst werden, die in die Stirnfäche des Schraubenkopfes 2 münden. In diese Ausnehmungen 3 können axiale Fortsätze 5 eines Eindrehwerkzeuges 6 (siehe Fig. 8) stirnseitig axial eingeschoben werden. Das Eindrehmoment wird in weiterer Folge auf die Seitenflächen 4 der Ausnehmungen 3 ausgeübt. Aufgrund der erfindungsgemäßen Ausführung des Schraubenkopfes 2 kann das maximale Endrehmoment jedoch erhöht werden, da die Festigkeit des Schraubenkopfes 2 durch Wegnahme einer geringstmöglichen Menge an Knochenmaterial erhöht wird. Zudem wird das Eindrehmoment auf kinematisch günstige Weise im äußeren Umfangsbereich des Schraubenkopfes 2 eingebracht. Das maximale Eindrehmoment kann auf diese Weise ausreichend erhöht werden, um auch eine Anwendung von Knochenschrauben als Zug- oder Kompressionsschrauben zu ermöglichen. Zudem wird sogar die Ausführung eines selbstschneidenden Gewindes zur Anwendung im spongiösen Knochenbereich möglich, sodass die Knochenschraube aus kortikalem Knochenmaterial somit in der Lage ist im spongiösen Knochen verwendet zu werden, ohne vorher ein Gewinde im spongiösen Knochen vorschneiden zu müssen.

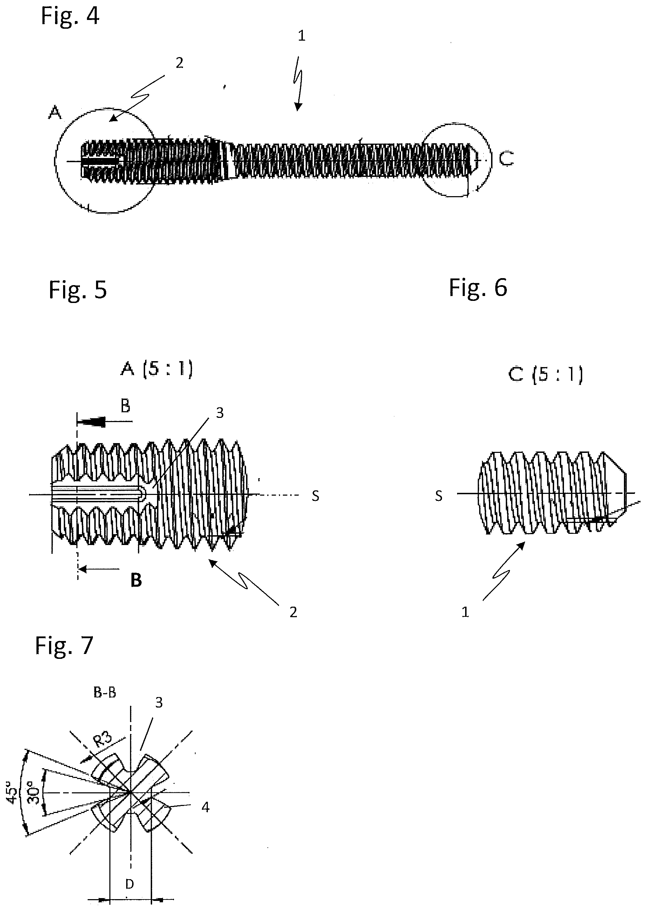

Die Fig. 4-7 zeigen eine weitere Ausführungsform eines erfindungsgemäßen Knochentransplantats, bei der das über Schraubenschaft 1 und Schraubenkopf 2 verlaufende Außengewinde zwei Abschnitte unterschiedlicher Gewindesteigung und Gewindeaußendurchmesser aufweist. Der Schraubenkopf 2 liegt dabei im Bereich mit dem größeren Gewindeaußendurchmesser, der beispielsweise eine Gewindesteigung von 0.8 mm aufweist. Der Bereich mit dem geringeren Gewindeaußendurchmesser weist beispielsweise eine Gewindesteigung von 1 mm auf. Eine solche Ausführungsform eignet sich insbesondere zur Ausführung einer Zug- oder Kompressionsschraube. Die beiden Abschnitte liegen dabei in ihrer chirurgischen Anwendung jeweils in den zu verbindenden Knochenbereichen. Aufgrund der unterschiedlichen Gewindesteigung werden bei einem bestimmten Drehwinkel, der freilich für beide Abschnitte gleich ist, die zu verbindenden Knochenbereiche aneinander gepresst.

Zur Ausführung einer Schraube mit selbstschneidendem Gewinde wird vorgeschlagen, dass das über Schraubenschaft 1 und Schraubenkopf 2 verlaufende Außengewinde zwei Abschnitte mit unterschiedlichem Gewindeaußendurchmesser aufweist. Bei einer solchen Schraube kann im Zuge der operativen Osteosynthese zunächst ein Kernloch für den größeren Gewindeaußendurchmesser in einem ersten Knochenteil vorgefertigt werden, und durch das größere Kernloch hindurch ein Kernloch für den geringeren Gewindeaußendurchmesser in einem zweiten Knochenteil, der mit dem ersten Knochenteil verbunden werden soll. Die Schraube kann nun ohne vorher ein Gewinde in das jeweilige Kernloch einschneiden zu müssen mit dem Abschnitt mit dem geringeren Gewindeaußendurchmesser durch das Kernloch für den größeren Gewindeaußendurchmesser eingeführt werden, bis es in das Kernloch des zweiten Knochenteils eingedreht werden kann. In weiterer Folge tritt die Schraube mit ihrem Abschnitt mit größerem Gewindeaußendurchmesser in das Kernloch des ersten Knochenteils ein und schneidet sich dort selbsttätig das Gewinde.

In den Fig. 8-10 wird eine mögliche Ausführungsform eines Eindrehwerkzeugs für das erfindungsgemäße Knochentransplantat dargestellt. Es weist vier von einem zylindrischen Schaft axial abstehende Fortsätze 5 auf, die stirnseitig in die Ausnehmungen 3 des Knochentransplantats axial eingeschoben werden können, bis sie mit großem Reibschluss in die Ausnehmungen 3 eingreifen. Der Reibschluss wird dabei durch die von der Außenmantelfläche in Richtung der Schraubenkopfachse S konvex verlaufenden Seitenflächen 4 der Ausnehmungen 3 erhöht, sowie durch die sich stetig in radialer Richtung verringernde Tiefe der Ausnehmungen 3 an ihrem der Stirnfläche abgewandten axialen Ende.

Aufgrund der erfindungsgemäßen Ausführung des Schraubenkopfes 2 wird die Einleitung des Eindrehmoments optimiert, also die Übertragung des über ein Eindrehwerkzeug auf den Schraubenkopf 2 ausgeübten Drehmoments. Die erfindungsgemäßen Ausnehmungen 3 gestatten dabei auch ein Fluchten des äußeren Schraubenkopfdurchmessers mit dem äußeren Durchmesser des Schraubenschaftes 1 sowie mit dem äußeren Durchmesser des Eindrehwerkzeuges und ermöglichen dadurch auch neue chirurgische Einsatzgebiete wie beispielsweise bei der intramedullären Schienung.

## Patentansprüche

1. Knochentransplantat aus einem kortikalen Knochenmaterial mit einem Schraubenschaft (1), der mit einem Außengewinde versehen ist, sowie einem Schraubenkopf (2) zur Einleitung eines Eindrehmoments, wobei der Schraubenkopf (2) eine um eine Schraubenkopfachse (S) rotationssymmetrische Außenmantelfläche aufweist, die mit einem Außengewinde versehen ist, sowie zumindest zwei um die Schraubenkopfachse (S) verteilt angeordnete, in Richtung der Schraubenkopfachse (S) axial verlaufende und in die Stirnfläche des freien Endes des Schraubenkopfes (2) mündende Ausnehmungen (3) zur Aufnahme eines Eindrehwerkzeuges aufweist, wobei die Ausnehmungen (3) jeweils durch sich von der Außenmantelfläche in Richtung der Schraubenkopfachse (S) erstreckende Seitenflächen (4) gebildet werden, die in einem konkav ausgeführten achsnahen Flächenabschnitt ineinander übergehen, **dadurch gekennzeichnet, dass** die von der Außenmantelfläche in Richtung der Schraubenkopfachse (S) verlaufenden Seitenflächen (4) der Ausnehmungen (3) konvex ausgeführt sind.

2. Knochentransplantat nach Anspruch 1, **dadurch gekennzeichnet, dass** vier symmetrisch um die Schraubenkopfachse (S) verteilt angeordnete Ausnehmungen (3) vorgesehen sind.

3. Knochentransplantat nach Anspruch 1 oder 2, **dadurch gekennzeichnet, dass** die Ausnehmungen (3) an ihrem der Stirnfläche abgewandten axialen Ende einen Bereich aufweisen, in dem sich die in radialer Richtung gemessene Tiefe der Ausnehmungen (3) stetig verringert.

4. Knochentransplantat nach einem der Ansprüche 1 bis 3, **dadurch gekennzeichnet, dass** das über Schraubenschaft (1) und Schraubenkopf (2) verlaufende Außengewinde zwei Abschnitte unterschiedlicher Gewindesteigung aufweist.

5. Knochentransplantat nach einem der Ansprüche 1 bis 4, **dadurch gekennzeichnet, dass** das über Schraubenschaft (1) und Schraubenkopf (2) erlaufende Außengewinde zwei Abschnitte mit unterschiedlichem Gewindeaußendurchmesser aufweist.

6. Knochentransplantat nach einem der Ansprüche 1 bis 5, **dadurch gekennzeichnet, dass** der Schraubenschaft (1) zylindrisch ausgeführt ist.

7. Knochentransplantat nach einem der Ansprüche 1 bis 5, **dadurch gekennzeichnet, dass** der Schraubenschaft (1) kegelstumpfförmig ausgeführt ist.

8. Knochentransplantat nach einem der Ansprüche 1 bis 7, **dadurch gekennzeichnet, dass** der Schraubenschaft (1) auf seinem dem Schraubenkopf (2) abgewandten, freien Ende eine zur Schraubenkopfachse (S) senkrechte Stirnfläche aufweist.

9. Knochentransplantat nach Anspruch 8, **dadurch gekennzeichnet, dass** die zur Schraubenkopfachse (S) senkrechte Stirnfläche des Schraubenschaftes (1) mit einer die Stirnfläche querenden Nut versehen ist, die an ihren gegenüberliegenden Seiten jeweils in die Außenmantelfläche des Schraubenschaftes (1) mündet.

10. Instrumentensatz mit zumindest einem Knochentransplantat nach einem der Ansprüche 1 bis 9 sowie zumindest einem Eindrehwerkzeug für das Knochentransplantat, das einen stabförmigen Kopplungsabschnitt aufweist, der mit zumindest zwei von einem freien Ende des Kopplungsabschnittes axial abstehenden Fortsätzen (5) versehen ist, wobei die zumindest zwei Fortsätze (5) in ihrer Außenquerschnittskontur zum reibschlüssigen Eingriff in die Ausnehmungen (3) ausgebildet und bestimmt sind.

## Claims

1. Bone graft made of a cortical bone material having a screw shank (1) which is provided with an external thread and a screw head (2) for introducing a torque, wherein the screw head (2) has an outer jacket surface which is rotationally symmetrical about a screw head axis (S) and is provided with an external thread, and has at least two recesses (3) which are arranged distributed about the screw head axis (S), extend axially in the direction of the screw head axis (S) and open into the end face of the free end of the screw head (2), for receiving an insertion tool, wherein the recesses (3) are each formed by side faces (4) which extend from the outer jacket surface in the direction of the screw head axis (S) and merge into one another in a surface section close to the axis which is of concave design, **characterized in that** the side surfaces (4) of the recesses (3) extending from the outer jacket surface in the direction of the screw head axis (S) are of convex design.

2. Bone graft according to claim 1, **characterized in that** four recesses (3) are provided which are distributed symmetrically about the screw head axis (S).

3. Bone graft according to claim 1 or 2, **characterized in that** the recesses (3) have at their axial end remote from the end face a region in which the depth of the recesses (3) measured in the radial direction is continuously reduced.

4. Bone graft according to one of claims 1 to 3, **characterized in that** the external thread extending over the screw shank (1) and screw head (2) has two sections of different thread pitch.

5. Bone graft according to one of claims 1 to 4, **characterized in that** the external thread running via the screw shank (1) and screw head (2) has two sections with different external thread diameters.

6. Bone graft according to one of claims 1 to 5, **characterized in that** the screw shank (1) is of cylindrical design.

7. Bone graft according to one of claims 1 to 5, **characterized in that** the screw shank (1) is of frustoconical design.

8. Bone graft according to one of claims 1 to 7, **characterized in that** the screw shank (1) has on its free end remote from the screw head (2) an end face perpendicular to the screw head axis (S).

9. Bone graft according to claim 8, **characterized in that** the end face of the screw shank (1) perpendicular to the screw head axis (S) is provided with a groove which crosses the end face and which opens on its opposite sides in each case into the outer jacket surface of the screw shank (1).

10. Instrument set having at least one bone graft according to one of claims 1 to 9 and at least one insertion tool for the bone graft which has a rod-shaped coupling section which is provided with at least two extensions (5) projecting axially from a free end of the coupling section, wherein the at least two extensions (5) are designed and intended in their outer cross-sectional contour for frictional engagement in the recesses (3).

## Revendications

1. Greffon osseux fait d'un matériau à base d'os cortical, avec un corps de vis (1) muni d'un filetage extérieur et avec une tête de vis (2) pour l'application d'un couple de vissage, dans lequel la tête de vis (2) présente une surface d'enveloppe externe symétrique en rotation autour d'un axe de tête de vis (S) qui est munie d'un filetage extérieur, et lequel la tête de vis (2) présente au moins deux évidements (3) répartis autour de l'axe de tête de vis (S), orientés dans le sens axial dans le sens de l'axe de tête de vis (S) et débouchant dans la face d'extrémité de l'extrémité libre de la tête de vis (2) pour recevoir un outil de vissage, lesquels évidements (3) sont formés chacun par des surfaces latérales (4) qui s'étendent à partir de la surface d'enveloppe extérieure dans la direction de l'axe de tête de vis (S) et qui se raccordent l'une à l'autre dans une section de surface proche de l'axe de forme concave, **caractérisé en ce que** les surfaces latérales (4) des évidements (3) qui s'étendent à partir de la surface d'enveloppe extérieure vers l'axe de tête de vis (S) ont une forme convexe.

2. Greffon osseux selon la revendication 1, **caractérisé en ce que** quatre évidements (3) répartis de façon symétrique autour de l'axe de tête de vis (S) sont prévus.

3. Greffon osseux selon la revendication 1 ou 2, **caractérisé en ce que** les évidements (3) présentent à leur extrémité axiale opposée à la face d'extrémité une zone dans laquelle la profondeur des évidements (3) mesurée dans le sens radial diminue.

4. Greffon osseux selon l'une des revendications 1 à 3, **caractérisé en ce que** le filetage extérieur passant sur le corps de vis (1) et la tête de vis (2) présente deux sections ayant un pas de filetage différent.

5. Greffon osseux selon l'une des revendications 1 à 4, **caractérisé en ce que** le filetage extérieur qui s'étend sur le corps de vis (1) et la tête de vis (2) présente deux sections ayant un diamètre extérieur de filetage différent.

6. Greffon osseux selon l'une des revendications 1 à 5, **caractérisé en ce que** le corps de vis (1) a une forme cylindrique.

7. Greffon osseux selon l'une des revendications 1 à 5, **caractérisé en ce que** le corps de vis (1) a une forme tronconique.

8. Greffon osseux selon l'une des revendications 1 à 7, **caractérisé en ce que** le corps de vis (1) présente à son extrémité libre tournée à l'opposé de la tête de vis (2) une face d'extrémité perpendiculaire à l'axe de tête de vis (S).

9. Greffon osseux selon la revendication 8, **caractérisé en ce que** la face d'extrémité du corps de vis (1) perpendiculaire à l'axe de tête de vis (S) est munie d'une rainure qui la traverse et débouche sur chacun de ses côtés opposés dans la surface d'enveloppe extérieure du corps de vis (1).

10. Jeu d'instrument comprenant au moins un greffon osseux selon l'une des revendications 1 à 9 et au moins un outil de vissage pour le greffon osseux, qui présente une section de couplage en forme de barre munie d'au moins deux saillies (5) qui dépassent dans le sens axial d'une extrémité libre de la section de couplage, les au moins deux saillies (5) ayant un contour en section extérieur conformé et étudié pour se mettre en prise par friction dans les évidements (3).
